# EUROPÄISCHE PATENTANMELDUNG

(11) **EP 2 189 426 A1**
(43) Veröffentlichungstag der Anmeldung: **26.05.2010**
(21) Anmeldenummer: 10000497.7
(22) Anmeldetag: 25.05.2005
(51) Int. Cl.: C03C 12/00, C03B 19/10

(54) **Verfahren zur Herstellung eines Glas- oder Glaskeramikpulvers in Form von Nanopartikeln**

(30) Priorität: 29.05.2004 DE 102004026433
(62) Teilanmeldung aus: 05744811.0
(71) Anmelder: Schott AG, 55122 Mainz (DE)
(72) Erfinder: Zimmer, José, 66679 Losheim am See (DE); Daimer, Johann, 84051 Oberkrain (DE); Rindt, Matthias, 65795 Hattersheim (DE); Kessler, Susanne, 84030 Ergolding (DE); Besinger, Jörn, 84032 Landshut (DE); Seneschal-Merz, Karine, 55118 Mainz (DE)
(74) Vertreter: Sawodny, Michael-Wolfgang

(57) **Zusammenfassung**

Die Erfindung betrifft ein Verfahren zur Herstellung eines Glas- oder Glaskeramikpulvers in Form von Nanopartikeln, umfassend wenigstens ein Multikomponentenglas mit mindestens drei Elementen, mit wenigstens den Schritten:
- Verdampfen aller Ausgangsmaterialien des Multikomponentenglases in einem Plasma,
- Abscheiden der verdampften Stoffe, wobei sich diese im glasartigen Zustand organisieren oder in amorpher Form erhalten werden,

wobei das Multikomponentenglas als Netzwerkbildner SiO₂ und/oder B₂O₃ und/oder P₂O₅ zwischen 30 und 95 Gew.-%, bevorzugt zwischen 30 und 80 Gew.-%, insbesondere zwischen 40 und 75 Gew.-%, am bevorzugtesten zwischen 50 und 70 Gew.-% enthält.

## Beschreibung

Gegenstand der Erfindung sind Multikomponentengläser mit mindestens drei (3) Elementen mit einer mittleren Partikelgrösse kleiner 1 µm, vorzugsweise kleiner 0,1 µm, besonders bevorzugt kleiner 10 nm. Gläser mit einer derartigen mittleren Partikelgrösse werden auch als Nanopulver bezeichnet. Neben den Multikomponentengläsern umfasst die Anmeldung auch Verfahren zur Herstellung solcher Gläser sowie deren Verwendung.

Anorganische Nanopulver sind für nicht kristalline Zusammensetzungen für SiO₂ und für kristalline Zusammensetzungen wie z. B. TiO₂ oder ZnO bekannt. Betreffend SiO₂-Nanopulver wird auf das Produkt Aerosil® der Degussa verwiesen.

Des Weiteren sind CeO-Nanopartikel für Poliersuspensionen z. B. von Fa. Nanophase (USA) sowie ZrO₂ -Nanopartikel oder Al₂O₃ Nanopartikel von der Fa. Nanogate (Deutschland) bekannt.

Metallische Nanopartikel sind z. B. für Silber und Silberlegierung bekannt. Eingesetzt werden derartige Nanopartikel beispielsweise als antimikrobielle Wirkstoffe z. B. für Polymere.

Pulver aus metallischen Nanopartikeln können auch zum Bonden im Bereich der Elektronik eingesetzt werden. Das Bonden ist gemäß H. D. Junge, A. Möschwitz, "Elektronik", VCH-Verlag 1993, S. 89 ein Schweißverfahren zum Kontaktieren von elektronischen Elementen beispielsweise auf einer integrierten Schaltung. Durch die Verwendung von metallischen Nanopulvern werden die Bondingtemperaturen stark herabgesetzt.

Neben den oben genannten Nanopartikeln sind auch Hydroxylapatit-Nanopartikel beispielsweise von der Firma BASF (Deutschland) bekannt geworden, die im Bereich der Mundpflege, Zahnhygiene, d.h. im Bereich der Oral Care-Anwendungen verwendet werden.

Wie aus dem zuvor beschriebenen Stand der Technik hervorgeht sind vor allem keramische Nanopartikel mit 2 Elementen, bestehend in der Regel aus einem Metall und einem Sauerstoff, bekannt geworden.

Als Nanopartikel in der glasigen Phase sind ausschließlich Zwei-Element-Systeme bestehend aus einer einzigen Komponente, nämlich reinen SiO₂-Partikeln bekannt. Nachteilig an derartigen reinen SiO₂-Systemen ist, dass sie auf Grund ihrer geringen chemischen Variabilität auch keine besonders breite Variation in den Materialeigenschaften aufweisen. Dies betrifft insbesondere optische, chemische, physikalische und mechanische Eigenschaften.

Gläser mit bioaktiver und teilweise auch antimikrobieller Wirkung werden bei LL. Hensch, J. Wilson, An Introduction to Bioceramics, World Scientific Publ., 1993, als Bioglas beschrieben. Derartiges Bioglas zeichnet sich durch die Bildung von Hydroxylappatitschichten in wässrigen Medien aus. Schwermetallfreie Alkali-Erdalkali-Silicat-Gläser mit antimikrobiellen Eigenschaften werden in den Anmeldungen WO 01/04252 und WO 01/03650 beschrieben.

Gläser mit antimikrobieller Wirksamkeit sind aus den nachfolgenden Patentanmeldungen WO03/018495, WO03/18498, WO03/18499, WO03/050052, WO03/062163, WO03/018496 bekannt geworden. Die in diesen Schriften beschriebenen Glaspulver wurden in Form von Mahlung beispielsweise in wässrigen Medien erhalten. Durch eine Mahlung wie in obigen Schriften beschrieben, können keine Glaspulver mit einer mittleren Partikelgrösse, die der von Nanopartikeln entspricht, erhalten werden.

Gläser, die im Dentalbereich Verwendung finden, sogenannte Dentalgläser, sind aus der DE 4323143, US 5,641,347, DE 4443173 sowie der EP 0997132 bekannt geworden.

Gläser und insbesondere Glaskeramiken, die sich durch eine geringe oder sogar durch eine Nullausdehnung auszeichnen, sind in der DE 19907038 und der US 5070045 gezeigt.

Die aus dem Stand der Technik bekannten Nanopartikel werden in einer Vielzahl von Gebieten eingesetzt. So ist die Anwendung von Nanopartikeln in kosmetischen Sonnenschutzformulierungen in der US20040067208 beschrieben

Die Oberflächenbehandlungen von Nanopartikeln sowie deren Drucken werden in der US20040052957 beschrieben.

Die Herstellung von Nanopartikeln sowie kratzfeste Beschichtungen mit Nanopartikeln werden in der DE0001022009A1 beschrieben.

Die DE000069600059 beschreibt die Verwendung von TiO₂-Nanopartikeln in Sonnenschutzmitteln.

Die US20040042953 beschreibt die Verwendung von Nanopartikeln in WC-Pulvern, wobei die mittlere Partikelgrösse zwischen 10 und 20nm schwankt. Hergestellt werden diese Nanopartikel über Gasphasenreaktionen.

Aus der US20030148282 ist die Verwendung von Nanopartikeln zur Detektion von Nukleinsäure bekannt.

Die US20030064532 beschreibt die Verwendung von Halbleiter-Nanopartikein im Bereich Luminiszenz und der optischen Datenspeicherung.

Die Herstellung beispielsweise von Silber- oder Silberlegierungsnanopulver geschieht über PVD-Verfahren.

Beispielsweise ist aus der US 4642207 ein PVD Plasmabogen-Verfahren zur Herstellung von Nanopartikeln über Verdampfung und Kondensation bekannt geworden.

Auch aus der US 5874684 ist ein Verfahren zur Herstellung von nanokristallinem Material bekannt geworden. Hierfür werden binäre Oxide als Rohmaterialien eingesetzt. Zur Herstellung unterschiedlicher Stoffe können verschiedene Atmosphären eingesetzt werden.

Aufgabe der Erfindung ist es ein Mehrkomponenten-Glaspulver zur Verfügung zu stellen, das sich dadurch auszeichnet, dass es in einer Vielzahl von Gebieten verwandt werden kann und gegenüber herkömmlichen Glaspulvern eine verbesserte Aktivität aufweist.

Gelöst wird die Aufgabe durch ein Glaspulver, welches Multikomponentengläser mit mindestens 3 Elementen aufweist, wobei die mittlere Partikelgrösse des Glaspulvers kleiner als 1 µm, bevorzugt kleiner 0,1 µm, noch bevorzugter kleiner 50 nm, besonders bevorzugt kleiner 10 nm aufweist.

In einer bevorzugten Ausführungsform weist das Glas mehr als 4, besonders bevorzugt mehr als 5, ganz besonders bevorzugt mehr als 6 Elemente auf.

In dieser Anmeldung wird als Komponente eines oxidischen Glases die oxidische Komponente verstanden, also beispielsweise SiO₂ oder B₂O₃. Unter dem Element in einer Glaszusammensetzung wird das einzelne Element, also Si oder B oder O verstanden. Ein Mehrkomponentenglas ist also ein Glas, welches beispielsweise als Komponenten SiO₂ und B₂O₃ umfasst. Ein Glas, das als Komponenten SiO₂ und B₂O₃ umfasst, weist insgesamt drei Elemente auf. Es würde sich also im Sprachgebrauch dieser Anmeldung um ein 2-Komponenten-Glas mit 3 Elementen handeln.

Gemäß der Erfindung umfassen die Glaspulver mit einer Partikelgrösse kleiner 1 µm, die auch als Nanogläser bezeichnet werden, als Netzwerkbildner SiO₂ und/oder B₂O₃ und/oder P₂O₅. Der Anteil des Netzwerkbildners bzw. der Summe der Netzwerkbildner, falls das Multikomponentenglas mehr als einen Netzwerkbildner umfasst, liegt bevorzugt zwischen 30-95 Gew%, vorzugsweise zwischen 30 und 80 Gew%, insbesondere zwischen 40 und 75 Gew%, am bevorzugtesten zwischen 50 und 70 Gew%. Gemäss der Hauptnetzwerkbildner können die Gläser in die Gruppe der Silikat-, Borat- oder Phosphat-Gläser eingeteilt werden.

Alkaliionen, wie z. B. Na, K, Li, Cs, können in die Glaszusammensetzung als Netzwerkwandler eingebracht werden. Die Konzentration der Alkalien liegen in der Summe zwischen 0 und 50 Gew%, bevorzugt zwischen 0 und 30 Gew%. Die Alkalien können auch der Einstellung der Reaktivität des Glases dienen, da durch die Alkalien das Glasnetzwerk gezielt unterbrochen werden kann. Beispielsweise können in die Glasmatrix eingebrachte Biozide Ionen wie z. B. Zn oder Ag leichter abgegeben werden.

Neben oder statt den Alkalien können die Erdalkaliionen, wie z. B. Mg, Ca, Sr, Ba, in Summe zwischen 0 und 50 Gew.-% vorliegen. Auch die Erdalkaliionen wirken als Netzwerkwandler und dienen der Einstellung der Reaktivität des Glases. Das Ca nimmt eine besondere Rolle ein. Bei speziellen bioaktiven Gläsern kann durch Vorhandensein von Ca eine Mineralschicht auf der Partikeloberfläche in wässrigen Medien ausbildet werden, die sogenannte Hydroxylapatit-Schicht. Die Multikomponentengläser können des weiteren Aluminiumoxid umfassen. Aluminiumoxid beeinflusst massgeblich die chemische Stabilität sowie die Kristallisationsstabilität der Gläser. Die Al₂O₃ Konzentration liegt bevorzugt zwischen 0 Gew% und 25 Gew%.

Neben den Netzwerkkomponenten kann das Glas Zinkoxid als eine wesentliche Glaskomponente umfassen. Die Zn-lonen des Glases können freigesetzt werden und zu einer antimikrobiellen Wirkung führen, die durch Alkali- oder Erdalkaliionen noch unterstützt wird. Die ZnO-Konzentration liegt üblicherweise in der Ausgangszusammensetzung der Rohstoffe zwischen 0-25 Gew%. Ausserdem kann Zink die chemische Beständigkeit der Gläser verbessern.

Die Multikomponentenmgläser können auch Titanoxid und/oder Zirkonoxid umfassen. Mit Hilfe dieser Zusätze kann gezielt die Brechzahl des Glaspulvers eingestellt werden. Insbesondere der Zusatz von TiO₂ kann auch zur UV-Blockung eingesetzt werden.

Falls die Nanopulver Glaskeramiknanopulver sind, können Zusätze von TiO₂ oder ZrO₂ als Keimbildner dienen.

Des Weiteren ist mit TiO₂ oder ZrO₂ eine Einstellung der chemischen Beständigkeit der Nanopulver möglich.

Die hydrolytische Beständigkeit kann insbesondere durch die Zugabe von ZrO₂ verbessert werden, was insbesondere bei hygroskopischen Nanopulvern von Bedeutung ist. Neben der Brechzahlanpassung können TiO₂ und ZrO₂ auch zur Einstellung des E-Moduls genutzt werden.

Bevorzugt liegt die Konzentration von TiO₂ zwischen 0 und 25 Gew% und die Konzentration von ZrO₂ liegt zwischen 0 Gew% und 30 Gew%.

Zur Feinanpassung der Brechzahl kann das Nanoglaspulver Tantal- und/oder Wolframoxid umfassen.

Neben oder statt Zn können zur Erzielung von antimikrobieller Wirksamkeit Ag, Cu, I im Glas enthalten sein. In Summe ist die Konzentration von

Ag₂O, CuO, ZnO, I kleiner 15 Gew%, bevorzugt kleiner 10; noch bevorzugter kleiner 5 Gew %.

Auch Edelmetalle wie Au, Pt können in metallischer oder oxidischer Form bis kleiner 10 Gew% vorzugsweise kleiner 5 Gew% am bevorzugtesten kleiner 2 Gew % enthalten sein.

Farbgebende Ionen wie z. B. Cr, Mn, Ni, V, Ce, Fe, V, Co können in Summe (Oxid) bis zu 10 Gew% vorliegen.

Selten Erd Ionen wie z. B. Eu, Ce, Sm, Nd, Er, Sm, Yb, können als Dotierung in üblichen Konzentrationen eingebracht werden.

Fluor kann in den Gläsern als Schmelzhilfsmittel enthalten sein.

Oxide der Elemente Nb, La, Pb und Bi dienen in erster Linie der Brechzahl bzw. Dispersionseinstellung.

Die Zugabe von Elemente wie z. B. Ba, Cs, La ermöglicht es eine hohe Radioopazität einzustellen.

Auch Läutermittel, wie z. B. SnO, As₂O₃, Sb₂O₃, können in der üblichen Konzentrationen in den Nanoglaspulvern enthalten sein, mit Ausnahme der Nanogläser, die in dentalen, medizinischen und kosmetischen Anwendungen Verwendung finden.

Die zuvor erwähnten Metalle Au, Ag, Pt, Cu können nicht nur in oxidischer, sondern auch in metallischer Form in der Glasmatrix vorliegen.

Radioaktive Elemente können ebenfalls zugesetzt werden.

In speziellen Ausführungsformen können auch Nitride oder Oxidnitride als Ausgangsmaterialien eingesetzt werden und darüber entsprechende Nitrid oder Oxinitrid-Nanogläser erhalten werden. Der Vorteil von Nitrid- oder Oxinitridnanogläser sind die besseren mechanischen Eigenschaften als bei oxidischen Gläsern.

Wie oben angegeben haben die erfindungsgemäßen Nanopulver mittlere Korngrössen kleiner 1 µm, bevorzugt kleiner 200 nm, insbesondere bevorzugt kleiner 100 nm, noch bevorzugter kleiner 50 nm, am bevorzugtesten kleiner 20 nm. In einer besonderen Ausführungsform werden Korngrössen kleiner 5 nm verwendet. In speziellen Ausführungsformen können die Nanopartikel kleiner 2 nm sein.

Die BET-Oberfläche herkömmlicher anorganischer Füllstoffe bei Dentalmaterialien liegt z. B. zw. 4 und 65 m²/g.

Im Gegensatz hierzu sind die BET-Oberfächen der Nanopartikel grösser als 50 m²/g, bevorzugt grösser als 100m²/g, noch bevorzugt grösser als 500 m²/g, am bevorzugtesten grösser als 900 m²/g.

Durch das hohe Oberflächen- zu Volumenverhältnis bei den erfindungsgemäßen Nanogläsern spielen die Oberflächeneigenschaften gegen über den Bulkeigenschaften eine zunehmend starke Rolle. Durch die hohe freie Oberfläche werden für den Fachmann auch bei an sich inerten Gläsern, wie es antimikrobielle Silikatgläser sind, überraschend hohe Reaktivitäten, insbesondere eine hohe Ionenabgaben z. B. in wässrigen Medien oder in organischen Verbindungen, hohe antimikrobielle Wirkung der Pulver erzielt.

Die Partikel können als Pulver und als Suspension zum Einsatz kommen.

Es können sowohl amorphe, phasenentmischte, kristallisierte Glas bzw. Glaskeramik-Nanopartikel eingesetzt werden. Unterschiedliche Phasen können bereits im primären Herstellungsprozess oder in einer Nachprozessierung erreicht werden.

Für die Verwendung als Füllstoff im Dentalbereich ist eine Modifikation der Oberfläche mit Organo-Silanen möglich und vorteilhaft, wie z. B. Methacryloxypropyl-tri-methoxy-Silan.

Die verwendeten Organo-Silane zeichnen sich besonders dadurch aus, dass sie sowohl an die Glasoberfläche anbinden können als auch über eine organische funktionelle Seitengruppe an ein organisches Harz binden können. Dadurch wird einerseits das Einformulieren in die organische Harzmatrix erleichtert und andererseits die mechanische Stabilität erhöht. Am weitesten verbreitet für Dentalanwendungen ist das 3-Methacryloxypropyltrimethoxysilan, besser bekannt unter dem Handelsnamen MEMO von der Degussa. Daneben gibt es eine Vielzahl weiterer funktioneller Seitengruppen, wie zum Beispiel Amino-, Glycidoxyl-, Mercapto-, Vinyl-, Alyl-Gruppen mit den entsprechenden Spacern.

Ionen der Elemente La, Ba, Sr, Y, Yb, Nb, Zr, Zn, dienen der Einstellung der Röntgensichtbarkeit von Dentalgläsern.

Die erfindungsgemäßen Nanopulvern, umfassend Multikomponentengläsern und -glaskeramiken können in den Bereichen Kosmetik z. B. als UV-Blocker für UV-A und/oder UV-B, Dentalfüller, Oral Care, optische Polymere, Sinterwerkstoffe, antimikrobielle Anwendungen, im medizinischen Bereich als Wirkstoff oder Wirkstoffträger, zur Wasserfilterung, -reinigung, aufbereitung, als Lotgläser; als Pigmente, zum Rapid Prototyping, was die sehr schnelle Herstellung von dreidimensionalen Strukturen beschreibt, in Brennstoffzellen, als Abrasivmaterialien, zur Katalyse, als UV-Schutz, in Polierprozessen, in Textilfasern, in Thermoplasten, in Farben und Lacken; in der Oberflächentechnologie, als Antihaft-, Antikratz-, Antireflex-, Antibeschlag-, einfach zu reinigende Schicht, zum Korrosionsschutz; im Bereich der keramischen Technologien, als Rohstoffe z. B. für Gläser oder als Glaskeramik, zur Kristallherstellung, zur Herstellung von optischen Glaskeramiken und optischen Keramiken sowie optischen Polymeren, in der Lasertechnologie, in der Drucktechnologie, in der Biotechnologie, als Fluoreszenzmarker, als Luminiszenzstoff, als Klebstoff, in Polymeren (z. B. Duromere, Plastomere, Monomere), als Kontaktlinsen in Folien, Druckpapier; Leuchtmittel, Kopiertechnik, Membranen verwendet werden.

Eine weitere Applikation stellt die Verwendung der Nanogläser im Bereich der Elektronik dar, beispielsweise als Glaslote zum Fügen oder als Passivierungsglas für Halbleiterbauelemente.

Die Herstellung der Nanopartikel erfolgt beispielsweise in einem PVD-Verfahren (Physical Vapor Deposition). Die PVD-Verfahren beschreiben eine Aufdampftechnik. Betreffend derartige PVD-Verfahren wird auf H. D. Junge und G. Müller, VDI-Lexikon Elektrotechnik, 1994, S.26 bis 27 oder VDI-Lexikon "Werkstofftechnik" VDI-Verlag, 1993, S. 810 - 811 sowie S. 5 - 6 verwiesen. Der Offenbarungsgehalt dieser Schriften wird vollumfänglich in die vorliegende Anmeldung mitaufgenommen. Bei PVD-Verfahren werden alle Stoffe des Glases in einem Plasma verdampft. Die verdampften Stoffe werden an einer kalten Fläche, beispielsweise einer Substratoberfläche abgeschieden und organisieren sich im glasigen Zustand neu. Es entstehen die erfindungsgemäßen Mehrkomponenten-Glas- oder Glaskeramik-Nanopartikel. Wie zuvor beschrieben kann neben Nanogläsern auch eine Nanoglaskermik oder ein Nanoglas, welches ein entmischtes System umfasst, auf diesem Weg hergestellt werden. Auch ist es möglich so hergestellte Nanogläser nachträglich einer Keramisierung zu unterwerfen. Auch die Herstellung von Nanopartikel mit Hilfe von Sol Gel-Verfahren ist möglich.

Neben den beschriebenen PVD-Verfahren können auch CVD-Verfahren verwendet werden. CVD-Verfahren (Chemical Vapor Deposition) beschreiben die chemische Abscheidung aus der Gasphase. Betreffend CVD-Verfahren wird auf das VDI-Lexikon "Werkstofftechnik" VDI-Verlag 1993, S.139 sowie S.5 - 6 verwiesen, dessen Offenbarungsgehalt vollumfänglich in den Offenbarungsgehalt der vorliegenden Anmeldung mit aufgenommen wird.

Ein weiteres Verfahren zur Herstellung von Nanopartikeln ist die Flammpyrolyse. Bei der Flammpyrolyse werden reaktive Gase in eine Flamme geleitet. In der Flamme werden die Nanopartikel synthetisiert und in kalten Regionen abgeschieden. Neben gasförmigen Rohstoffen können auch flüssige Rohmaterialien bei der Flammenpyrolyse eingesetzt werden.

Werden in den beschriebenen Verfahren, insbesondere im PVD-Verfahren nichtoxidisches Trägergas eingesetzt, so können Nitrid- oder Oxinitrid-Nanogläser hergestellt werden.

Zur Herstellung der beschriebenen Nanogläser oder Nano-Glaskeramiken eignen sich ganz besonders die oben beschriebenen PVD-Verfahren. Bei den PVD-Verfahren sind besonders die Plasmaverfahren, insbesondere Plasmaverfahren kombiniert mit Hochfrequenzverdampfung oder Elektronenverdampfung, geeignet. Die Plasmaverfahren zeichnen sich dadurch aus, dass die Verdampfung des Rohmaterials in einem Plasma erfolgt.

Als Ausgangsmaterialien werden in den im Stand der Technik bekannten PVD-Verfahren Metalle oder Metalloxide werden.

Besonders bevorzugt ist es aber zur Herstellung der erfindungsgemäßen Multikomponentengläser mit Partikelgrössen kleiner 1 µm als Ausgangsmaterialien aber bereits Multikomponentengläser zu verwenden. Werden Multikomponentengläser als Ausgangsmaterialien eingesetzt, so können unterschiedliche Multikomponentengläser in unterschiedlichen Gewichtsanteilen und Korngrössenverteilungen gemischt werden.

Durch die Verwendung von Multikomponentengläsern als Rohstoffe können bereits in einem Rohstoff geeignete Elementkombinationen zusammengestellt sein. Im PVD-Verfahren wird durch lokale Erwärmung des Multikomponentenglases als Rohstoff, dieser Rohstoff selektiv verdampft und die Rohstoffe dann als erfindungsgemäßes Glaspulver oder Glaskeramikpulver mit Partikelgrössen kleiner 1 µm wieder abgeschieden. Wie beschrieben werden die Ausgangsmaterialien beispielsweise in Stab- oder Pulverform in einen Rezipienten eingebracht und dort in einem Plasmabogen verdampft und in einem Gasstrom anschliessend die entsprechenden Nanopartikel abgeschieden.

Der Vorteil des PVD-Verfahrens ist, dass durch die schnellen Abkühlraten auch kristallisationsanfällige Gläser in amorpher Form abgeschieden werden können. Dies betrifft auch Gläser die unter Standardschmelzbedingungen nicht stabil produziert werden können und von denen somit über konventionelles Schmelzen und Mahlen kein amorphes Glaspulver erhalten werden kann.

Durch Einführung unterschiedlicher Reaktionsgase können Oberflächenmodifkationen sowie Gesamtzusammensetzungsmodifkationen erzielt werden. Beispielsweise können mit Hilfe oxidischer Trägergase oxidische Gläser abgeschieden werden, mit Hilfe nicht-oxidische Trägergase beispielsweise OxiNitrid-Gläser.

Aufgrund der sehr kleinen Partikelgrösse lassen sich die erfindungsgemäßen Gläser zur Spaltüberbrückung bei Bonding-Verfahren oder als Klebeverbindungen in optischen Applikationen, zur UV- oder IR-Absorption, zur Wärmedämmung, zur Lichtreflexion, als feuerresistenter Stoff, als Dichtstoff, als Glanzstoff, als Farbbrillianz-Stoff, sowie in der Elektrostatik verwenden.

Weitere Einsatzgebiete sind poröse Elektroden für Brennstoffzellen, Hartlote für Keramik-Metall-Verbindungen oder Tieftemperturlote. Hierbei insbesondere Lote im Bereich von Glas-Glas, Glas-Metall, Glass-Keramik oder Glas-Kristall Verbindungen.

Weiterhin können allgemein jeweils Gläser, Keramiken, Glaskeramiken, Kristalle, Metalle mit solchen Loten untereinander verbunden werden.

Auch können die erfindungsgemäßen Nanopartikel elektrophoretisch auf Oberflächen bzw. in porösen Körpern abgeschieden werden.

Die im Stand der Technik beschrieben anorganischen nicht metallischen Biozide können nur in relativ großen Partikelgrössen größer 1 µm hergestellt und eingesetzt werden. Sie haben daher eine geringere Wirksamkeit als organische Biozide.

Überraschenderweise kann durch die erfindungsgemäßen Nanopartikel die Reaktivität, insbesondere aber die antimikrobielle Wirksamkeit aussergewöhnlich stark gesteigert werden. Hierbei kommt nicht nur die höhere Verfügbarkeit der eingelagerten Wirkstoffe, wie z. B. Ag, Zn, Cu, zum Tragen, sondern die Glasoberfläche an sich mit einem entsprechenden Zetapotential bzw. lokal hohen pH-Werten. Die erhöhte Oberfläche generiert einen synergistischen zusätzlichen antimikrobiellen Effekt. Gegenüber den metallischen antimikrobiellen Nanopulvern beispielsweise Silber-Nanopulver ergibt sich der Vorteil dass die oxidischen Verbindungen wenig zur Verfärbung neigen und das Silber bereits in seine antimikrobielle wirksamen oxidierten Form vorliegt. Die Glas- oder Glaskeramik-Nanopartikel können von der Zusammensetzung so eingestellt werden, dass sie sich in wässrigen Systemen komplett auflösen.

Werden Nanopulver aus Nullausdehnungsmaterial erfindungsgemäß erhalten, so eignen sich diese besonders für die Sinterung und als Füllstoff. Insbesondere ist es möglich durch Sinterung derartiger Nanopulver Nullausdehnungsformkörper über die Sinterroute herzustellen. Mit Hilfe der erfindungsgemäßen Nanopartikel ist es möglich die Sintertemperatur zu erniedrigen und sehr hohe Enddichten mit sehr geringer Porosität zu erreichen, die sich durch eine geringe Streuung und hohe Transparenz auszeichnen.

Auch optische Gläser lassen sich aus den erfindungsgemäßen Nanopartikeln durch viskoses Sintern erhalten.

Die Nanogläser ergeben zusammengesetzt einen Sintergrünling. Aufgrund der Zusammensetzung des Sintergrünlinges aus einer Vielzahl von einzelnen Nanopartikeln wird eine extrem hohe Oberfläche in den Sintergrünling eingebracht. Aufgrund dieser extrem hohen Oberfläche können besondere Gefüge mit kleinsten Kristallitgrössen erzeugt werden. Die Kristallisation des Sintergrünlinges kann hierbei je nach Glastyp sowohl oberflächengesteuert oder volumengesteuert ablaufen. Ein weiterer Vorteil der extrem hohen Oberfläche der Grünlinge ist, dass Nanokristalle (sowohl bei Volumen als auch bei oberflächengesteuerter Kristallisation) in den gesinteren Vollmaterialien erzeugt werden. Dies ist ein Weg, um Sinterglaskeramiken mit Nanokristallen zu erzeugen.

Durch die hohe Oberflächenreaktivität können Nano-Glaspulver auch als Sinterhilfsmittel für hochschmelzende Materialien eingesetzt werden. Eine andere Anwendung ist der Einsatz beim Verschmelzen temperaturempfindlicher Materialien oder Halbfertigprodukte. Mit Hilfe der früh einsetzenden Oberflächenreaktion kann hier die Löttemperatur herabgesetzt werden.

Lotgläser aus Nanopartikeln insbesondere in Verbindung mit Lasersintern bzw. Laserlöten werden eingesetzt um möglichst niedrige Temperatur- und Spannungsbelastungen zu erzielen.

Ein weiterer Vorteil der erfindungsgemäßen Nanogläser liegt darin, dass im Gegensatz zu kristallinen keramischen Nanopartikeln Gläser in einem grossen Bereich in ihren optischen Lagen eingestellt werden können. Diese Möglichkeit der Einstellung betrifft beispielsweise die Transmission, Brechzahl, Dispersion und auch Teildispersion des Glases. Durch Mischungen von Polymeren mit Nanogläsern ist es möglich, Polymer-Glas-Komposite zu erhalten, bei denen die optischen Parameter sehr präzise eingestellt werden können. Durch die Variabilität der Glaschemie und entsprechender Oberflächenmodifikation die während und nach der Herstellung durchgeführt werden, können auch Eigenschaften wie die Dispergierfähigkeit eingestellt werden. Dies ist z. B. notwendig, wenn Nanopatikel in Monomeren dispergiert werden.

Da aufgrund der geringen Partikelgröße der Nanopartikel ein sehr hoher Füllgrad bis über 50 Gew% in die Monomere eingebracht werden kann, ohne die Viskosität des Monomers zu beeinflussen, kann durch den Einsatz von Nanopulvern aus Glas, welches beispielsweise im Brechungsindex an die Anwendung angepasst wird, ein hochgefülltes Polymeres mit geringem Polymerisationsschrumpf erzeugt werden. In den hochgefüllten Polymeren können dann optische Effekte, beispielsweise ein Tyndall-Effekt gezielt erzeugt oder auch vermieden werden.

Beim Einsatz farbiger Gläser ist es möglich, Polymere auch dann einzufärben, wenn handelsübliche keramische Pigmente nicht verwendet werden können und organische Farbstoffe aus Gründen der Toxikologie oder der chemischen, thermischen oder UV-Beständigkeit nicht eingesetzt werden sollen.

Ein weiteres Einsatzgebiet der erfindungsgemäßen Nanoglaspulver ist das sogenannte Rapid Prototyping, d.h. die Herstellung dreidimensionaler Prototypen beispielsweise auf dem Gebiet des Tissue Engineering also der Herstellung dreidimensionaler Implantatgerüste, die als Trägermaterialien für das Wachstum von Gewebezellen dienen.

Die Nanoglaspulver bzw. Nanoglaskeramikpulver sind wegen der hohen Biokompatibilität auch als Implantatmaterial, Beschichtungsmaterial für Implantate oder Trägersystem für Medikamente einsetzbar. Wegen der entzündungshemmenden bzw. antimikrobiellen Eigenschaften sind die erfindungsgemäßen Nanogläser oder Nanoglaspulver auch direkt als Wirkstoff einsetzbar. Alternativ ist es möglich, die Wirkstoffe in das Glas einzubringen oder die Wirkstoffe auf die Glasoberfläche aufzubringen. Derartige Systeme stellen dann sogenannte "Releasesysteme" dar.

Auch Komposit-Materialien z. B. aus LGA und/oder PGA bzw. deren Copolymere für Biomaterial insbesondere für Tissue engineering sind möglich. LGA und PGA sind bioresorbierbare Polymere.

Eine Verwendung im kosmetischen Bereich der erfindungsgemäßen Nanopartikel ist möglich. Insbesondere kann bei Anwendung im kosmetischen Bereich eine UVblockende und/oder lichtstreuende Wirkung hervorgerufen werden.

Auch die Herstellung von erfindungsgemäßen Glas- und/oder Glaskeramiknanopartikel mit antioxidativer, entzündungshemmender, antimikrobieller, remineralisierender Wirkung ist möglich. Werden bestimmte Stoffe zugegeben, so ist es möglich magnetische Nanopartikel beispielsweise für durchblutungsfördernde Behandlungen herzustellen.

Da die chemische Zusammensetzung der Gläser variiert werden kann, ist es möglich die mechanischen Eigenschaften der Nanopartikel aus Glas- oder Glaskeramik, wie z. B. Härte, E-Modul, Dichte, chemische Beständigkeit (z. B. gegen Wasser, Lauge u. Säuren) oder die elektrischen Eigenschaften, einzustellen und anzupassen. Neben der Partikelgrösse kann auch das Zetapotential durch Zusammensetzungs- und/oder Oberflächenmodifikationen angepasst werden.

Nachfolgend soll die Erfindung anhand von Ausführungsbeispielen näher beschrieben werden.

In Tabelle 1 sind Zusammensetzungen von Gläsern oder Ausgangsgläsern für Glaskeramiken in Gew.-% angegeben, aus denen mit den erfindungsgemäßen Verfahren Nanoglas- oder Nanoglaskeramikpartikel hergestellt werden können.

Beispielsweise beziehen sich bei PVD-Verfahren die angegebenen Glaszusammensetzungen gemäß Tabelle 1 auf die Glaszusammensetzungen der Ausgangsgläser, die mit Hilfe beispielsweise eines Elektronenstrahles verdampft werden können. Die Glaszusammensetzung der im PVD-Verfahren abgeschiedenen Nanoglas- oder der Nanoglaskeramikpartikel, stimmen bei entsprechender Verfahrensführung im wesentlichen mit den Zusammensetzungen der Ausgangsgläser überein.

**Tabelle 1: Glaszusammensetzungen**

| Beispiel | 1 | 2 | 3 | 4 | 5 | 6 | 7 | 8 | 9 | 10 |
|---|---|---|---|---|---|---|---|---|---|---|
| SiO₂ | 45 | 65 | 73,5 | 50,4 | 93 | 80 | 5 | 5 | | 45 |
| B₂O₃ | | 15 | 10,0 | 13,4 | | | 50 | 20 | | |
| P₂O₅ | 5 | | | | | | | | 55 | 5 |
| Na₂O | 25 | 17 | 6,6 | 0,1 | | | 10 | | 2,5 | 24 |
| Li₂O | | | | | | | | | 2,5 | |
| K₂O | | | 2,6 | | | | | | 2 | |
| CaO | 25 | | 0,6 | | | | | | | 24 |
| MgO | | | | | | | 5 | | | |
| SrO | | | | 0,3 | | | | | | |
| BaO | | | | 24,0 | | | | | | |
| Al₂O₃ | | 1 | 6,7 | 11,8 | | | | | 3 | |
| TiO₂ | | | | | 7 | | | 2,5 | | 2 |
| ZrO₂ | | | | | | 20 | | 2,5 | | |
| ZnO | | | | | | | | 20 | 35 | |
| La₂O₃ | | | | | | | | 35 | | |
| WO₃ | | | | | | | | 5 | | |
| Nb2O₅ | | | | | | | | 10 | | |
| PbO | | | | | | | 30 | | | |
| Ag₂O | | 2 | | | | | | | | |

Unter üblichen Läutermittel werden in dieser Anmeldung beispielsweise als Läutermittel Sn₂O₃,NaCl,As₂O₃, Sb₂O₃, As₂S₃, Sb₂S₃, verstanden, als übliche Mengen eines üblichen Läutermittels werden 0 - 4 Gew% der Gesamtzusammensetzung verstanden.

Im nachfolgenden sollen Ausführungsbeispiele für Nanoglaspulver und deren Verwendung gegeben werden.

Ausführungsbeispiel 1 bezieht sich auf ein Nanoglaspulver, das in eine Polymermatrix eingebracht und zu einer antimikrobiellen Wirkung des Polymer-Nanoglas-Komposit-Werkstoffes führt. Gemäß Ausführungsbeispiel 1 werden 0,1 Gew% Nanoglaspulver mit einer Partikelgrösse kleiner 1 µm gemäß Beispiel 2 in Tabelle 1 in eine Polystyrolmatrix eingearbeitet und zu Platten extrudiert. Die antimikrobielle Wirksamkeit der Oberfläche wird nach ASTM-Standard getestet. Es wird eine Reduktion der Testkeime (E. Coli, Candida Albicans) um mehr als 2 log-Stufen bestimmt.

Gemäß Ausführungsbeispiel 2 werden 0,1 Gew.-% bioaktives Nanoglaspulver mit Partikelgrößen kleiner 1 nm gemäß Beispiel 1 in Tabelle 1, um eine Formulierung für ein Deodorant eingearbeitet. Eine signifikante Schweißreduktion wird beobachtet.

In Ausführungsbeispiel 3 werden 50 Gew.-% des erfindungsgemäßen Nanoglaspulver in ein Dentalharz einformuliert. Typische Dentalharze sind in der EP 0475239 und darin zitierten Schriften beschrieben. Das Nanoglas des Glaspulvers hat eine Glaszusammensetzung gemäß Beispiel 4 in Tabelle 1. Die mittlere Partikelgröße ist kleiner als 1 µm.

In Ausführungsbeispiel 4 wird ein hochschmelzendes Glas (z. B. das Schott Glas mit Nummer 8330) mit Nanopulver als Beimischung gemischt, um die Sintertemperatur herabzusetzen.

Ausführungsbeispiel 5 betrifft ein Lotglas, bestehend aus 70 Vol% Nanoglaspulver mit einer Zusammensetzung gemäß Beispiel 9 in Tabelle 1 und einer Partikelgröße < 1 µm und 30 Vol% eines inerten Füllstoffes (z. B. Cordierit) zur Dehnungsanpassung. Das so erhaltene Nano-Kompositglaslot weist eine um 50°C geringere Einschmelztemperatur auf verglichen mit der gleichen Mischung des Ursprungsmaterials.

Ausführungsbeispiel 6 betrifft ein Polymer - Glaskomposit, bei dem ein Fluorpolymer jeweils mit 5,10,20 Gew% eines Nanopulvers, das die Glaszusammensetzung eines Bleisilikatglases mit einer Brechzahl n = 1,9 aufweist, versetzt wird. Je nach Anteil des Nanopulvers im Fluorpolymeren verschiebt sich die Brechzahl des Kompositwerkstoffes zu höheren Werten.

In Ausführungsbeispiel 7 wird 5 Gew.-% eines Nanoglaspulver mit Partikelgrößen kleiner 1 µm mit einer Glaszusammensetzung, die 2 Gew.-% TiO₂ umfasst, einer Sonnenmilch-Formulierung zugegeben, um eine UV-Blockung zu erreichen.

Die Erfindung umfasst Aspekte, die in den nachfolgenden Sätzen offenbart sind, die Teil der Beschreibung, aber nicht der Ansprüche gemäß der Entscheidung der Beschwerdekammer J15/88 darstellen:

### Sätze:

1. Glas- oder Glaskeramikpulver, umfassend Multikomponentengläser mit mindestens drei Elementen, **dadurch gekennzeichnet, dass** das Glas- oder Glaskeramikpulver eine mittlere Partikelgröße kleiner 200 nm sowie eine antimikrobielle Wirksamkeit aufweist.
2. Glas- oder Glaskeramikpulver, umfassend Multikomponentengläser mit mindestens drei Elementen, **dadurch gekennzeichnet, dass** das Glas- oder Glaskeramikpulver eine mittlere Partikelgröße kleiner 1 µm, vorzugsweise kleiner 0,1 µm, besonders bevorzugt kleiner 10 nm aufweist.
3. Glas- oder Glaskeramikpulver gemäß Satz 2, **dadurch gekennzeichnet, dass** die Nanopartikel eine BET-Oberfläche größer als 50 m²/g, bevorzugt größer als 100 m²/g, Nanoglaspulver und deren Verwendung, insbesondere Multikomponenten-Glaspulver mit einer mittleren Partikelgrösse kleiner 1 µm.
4. Glas- oder Glaskeramikpulver gemäß einem der Sätze 1 bis 4, **dadurch gekennzeichnet, dass** das Glas- oder Glaskeramikpulver mehr als drei Elemente umfasst.
5. Glas- oder Glaskeramikpulver gemäß einem der Sätze 1 bis 5, **dadurch gekennzeichnet, dass** das Glas- oder Glaskeramikpulver mehr als vier Elemente umfasst.
6. Glas- oder Glaskeramikpulver gemäß einem der Sätze 1 bis 5, **dadurch gekennzeichnet, dass** das Glas- oder Glaskeramikpulver mehr als fünf Elemente umfasst.
7. Glas- oder Glaskeramikpulver gemäß einem der Sätze 1 bis 6, **dadurch gekennzeichnet, dass** das Ausgangsmaterial zur Herstellung der Glas- oder Glaskeramikpartikel oder die Glas- oder Glaskeramikpulvernanopartikel selbst die nachfolgende Zusammensetzung (in Gew.-% auf Oxidbasis) umfasst:

| | |
|---|---|
| SiO₂ | 0-90 |
| B₂O₃ | 0-90 |
| P₂O₅ | 0-90 |
| N₂O | 0-30 |
| Li₂O | 0-30 |
| K₂O | 0-30 |
| CaO | 0-30 |
| MgO | 0-40 |
| CsO | 0-40 |
| BaO | 0-40 |
| SrO | 0-40 |
| Al₂O₃ | 0-30 |
| TiO₂ | 0-20 |
| ZnO | 0-30 |
| Nb₂O₃ | 0-40 |
| La₂O₃ | 0-40 |
| PbO | 0-70 |
| Bi₂O₃ | 0-70 |
| WO₃ | 0-30 |
| ZrO₂ | 0-40 |
| Yb₂O₃ | 0-40 |
| Y₂O₃ | 0-40 |
| F | 0-10 |
| Ag₂O | 0-5 |
| CuO | 0-10 |

wobei die Summe SiO₂+ B₂O₃+P₂O₅ größer 25 Gew.-% ist.
8. Glas- oder Glaskeramikpulver nach Satz 7, **dadurch gekennzeichnet, dass** die Zusammensetzung (in Gew.-% auf Oxidbasis) umfasst:

| | |
|---|---|
| SiO₂ | 50-80 |
| B₂O₃ | 0-15 |
| P₂O₅ | 0-20 |
| Na₂O | 0-10 |
| Li₂O | 0-15 |
| K₂O | 0-15 |
| CaO | 0-15 |
| MgO | 0-15 |
| BaO | 0-5 |
| SrO | 0-5 |
| Al2O₃ | 0-30 |
| TiO₂ | 0-10 |
| ZnO | 0-10 |
| ZrO₂ | 0-10 |
| F | 0-10 |

sowie übliche Läutermittel in üblichen Mengen.
9. Glas- oder Glaskeramikpulver gemäß Satz 7, **dadurch gekennzeichnet, dass** die Glaszusammensetzung 40 - 80 Gew.-% P₂O₅ enthält.
10. Glas- oder Glaskeramikpulver gemäß Satz 7, **dadurch gekennzeichnet, dass** die Glaszusammensetzung 30 - 80 Gew.-% B₂O₃ enthält.
11. Glas- oder Glaskeramikpulver nach Satz 7, **dadurch gekennzeichnet, dass** die Glaszusammensetzung 20 - 95 Gew.-% SiO₂ enthält.
12. Glas- oder Glaskeramikpulver nach einem der Sätze 7 bis 11, **dadurch gekennzeichnet, dass** die Glaszusammensetzung 4 - 30 Gew.-% CaO enthält.
13. Glas- oder Glaskeramikpulver nach einem der Sätze 7 bis 11, **dadurch gekennzeichnet, dass** die Glaszusammensetzung 4 - 30 Gew.-% Na₂O enthält.
14. Glas- oder Glaskeramikpulver nach einem der Sätze 7 bis 11, **dadurch gekennzeichnet, dass** die Glaszusammensetzung 40 - 80 Gew.-% SiO₂ und 5 - 50 Gew.-% B₂O₃ enthält.
15. Glas- oder Glaskeramikpulver nach einem der Sätze 1 bis 14, **dadurch gekennzeichnet, dass** die Glaszusammensetzung Ag, Cu, Zn, I enthält.
16. Glas- oder Glaskeramikpulver nach einem der Sätze 1 bis 15, **dadurch gekennzeichnet, dass** das Glas- oder Glaskeramikpulver entzündungshemmende Wirkung aufweist.
17. Glas- oder Glaskeramikpulver nach einem der Sätze 1 bis 16, **dadurch gekennzeichnet, dass** das Glas- oder Glaskeramikpulver mittels PVD-Verfahren hergestellt wird.
18. Verwendung von Glas- oder Glaskeramikpulvern nach einem der Sätze 1 bis 17 in einem oder mehreren der nachfolgenden Gebiete:
im Bereich Oral Care
im Bereich Dentalfüllstoffe
in medizinischen Produkten
in der Sintertechnologie
als antimikrobielle Wirkstoffe
als Füllstoff für Polymere
im Bereich Kosmetika
im Bereich Lotgläser
im Bereich Oberflächen
im Bereich Dentalkeramiken
im Bereich Medizinprodukte
als Passivierungsmittel mit Passivierungseigenschaften
mit einer organofunktionalisierten Oberfläche
im Bereich Infiltrationsgläser oder
im Bereich Photokatalyse.
19. Verwendung eines Glas- oder Glaskeramikpulvers, umfassend Multikomponentengläser mit mindestens drei Elementen und einer mittleren Partikelgröße kleiner 200 nm als Füllstoff im Dentalbereich.

## Patentansprüche

1. Verfahren zur Herstellung eines Glas- oder Glaskeramikpulvers in Form von Nanopartikeln, umfassend wenigstens ein Multikomponentenglas mit mindestens drei Elementen, mit wenigstens den Schritten:
- Verdampfen aller Ausgangsmaterialien des Multikomponentenglases in einem Plasma,
- Abscheiden der verdampften Stoffe, wobei sich diese im glasartigen Zustand organisieren oder in amorpher Form erhalten werden,
wobei das Multikomponentenglas als Netzwerkbildner SiO₂ und/oder B₂O₃ und/oder P₂O₅ zwischen 30 und 95 Gew.-%, bevorzugt zwischen 30 und 80 Gew.-%, insbesondere zwischen 40 und 75 Gew.-%, am bevorzugtesten zwischen 50 und 70 Gew.-% enthält.

2. Verfahren nach Anspruch 1, **dadurch gekennzeichnet, dass** als Gasstrom ein oxidisches Trägergas oder ein nicht-oxidisches Trägergas eingesetzt wird.

3. Verfahren nach Anspruch 1 oder 2, **dadurch gekennzeichnet, dass** zum Verdampfen ein Hochfrequenzverdampfen oder ein Elektronenverdampfen eingesetzt wird.

4. Verfahren nach einem der Ansprüche 1 bis 3, **dadurch gekennzeichnet, dass** als Ausgangmaterialien Metalle oder Metalloxide verwendet werden.

5. Verfahren nach einem der Ansprüche 1 bis 4, **dadurch gekennzeichnet, dass** durch lokale Erwärmung eines Multikomponentenglases als Rohstoff dieser selektiv verdampft und die Rohstoffe als Glaspulver oder Glaskeramikpulver mit Partikelgrößen kleiner 1 µm abgeschieden werden.

6. Verfahren nach einem der Ansprüche 1 bis 5, **dadurch gekennzeichnet, dass** durch die schnellen Abkühlraten des PVD-Verfahrens kristallisationsanfällige Gläser in amorpher Form und Gläser, die unter Standardschmelzbedingungen nicht stabil produziert werden können hergestellt werden.

7. Verfahren nach mindestens einem der vorangehenden Ansprüche, **dadurch gekennzeichnet, dass** die Glaszusammensetzung der im PVD-Verfahren abgeschiedenen Nanoglas- oder Nanoglaskeramikpartikel mit den Zusammensetzungen der Ausgangsgläser übereinstimmen, die die nachfolgende Zusammensetzung (in Gew.-% auf Oxidbasis) umfassen:
| | |
|---|---|
| SiO₂ | 0-90 |
| B₂O₃ | 0-90 |
| P₂O₅ | 0-90 |
| Na₂O | 0-30 |
| Li₂O | 0-30 |
| K₂O | 0-30 |
| CaO | 0-30 |
| MgO | 0-40 |
| CsO | 0-40 |
| BaO | 0-40 |
| SrO | 0-40 |
| Al2O₃ | 0-30 |
| TiO₂ | 0-20 |
| ZnO | 0-30 |
| Nb₂O₃ | 0-40 |
| La₂O₃ | 0-40 |
| PbO | 0-70 |
| Bi₂O₃ | 0-70 |
| WO₃ | 0-30 |
| ZrO₂ | 0-40 |
| Yb₂O₃ | 0-40 |
| Y₂O₃ | 0-40 |
| F | 0-10 |
| Ag₂O | 0-5 |
| CuO | 0-10 |
wobei die Summe SiO₂+ B₂O₃+P₂O₅ grösser 30 Gew% ist.

8. Verfahren nach Anspruch 6, **dadurch gekennzeichnet, dass** die Glaszusammensetzung 4 - 30 Gew% CaO enthält.

9. Verfahren nach Anspruch 6, **dadurch gekennzeichnet, dass** die Glaszusammensetzung 4 - 30 Gew% Na₂O enthält.

10. Verfahren nach Anspruch 6, **dadurch gekennzeichnet, dass** die Glaszusammensetzung Ag, Cu, Zn, I enthält.

11. Verfahren nach mindestens einem der vorangehenden Ansprüche, **dadurch gekennzeichnet, dass** die hergestellten Nanopartikel antioxidative, entzündungshemmende, antimikrobielle, remineralisierende Wirkung aufweisen.
